# DEMANDE DE BREVET EUROPEEN

(11) **EP 1 430 885 A1**
(43) Date de publication de la demande: **23.06.2004**
(21) Numéro de dépôt: 03292686.7
(22) Date de dépôt: 28.10.2003
(51) Int. Cl.: A61K 7/50, A61K 7/48

(54) **Compositions cosmétiques contenant un tensioactif amphothère et un agent nacrant et leurs utilisations**

(30) Priorité: 19.12.2002 FR 0216194
(71) Demandeur: L'OREAL, 75008 Paris (FR)
(72) Inventeur: Maubru, Mireille, 78400 Chatou (FR)
(74) Mandataire: Le Blainvaux Bellegarde, Françoise

(57) **Abrégé**

L'invention concerne de nouvelles compositions cosmétiques comprenant dans un milieu cosmétiquement acceptable au moins un agent opacifiant et/ou nacrant et au moins un tensioactif amphotère choisi parmi les composés alcoylamphohydroxyalkylsulfonates et leurs sels.

Cette association apporte des propriétés cosmétiques améliorées (légèreté, volume).

Ces compositions sont utilisées notamment pour le lavage et/ou le conditionnement des matières kératiniques telles que les cheveux ou la peau.

## Description

La présente invention concerne de nouvelles compositions cosmétiques comprenant dans un milieu cosmétiquement acceptable au moins un agent opacifiant et/ou nacrant et au moins un tensioactif amphotère choisi parmi les composés alcoylamphohydroxyalkylsulfonates et leurs sels.

Les agents nacrants et/ou opacifiants sont couramment utilisés dans les compositions cosmétiques en particulier les compositions détergentes telles que les shampooings afin de conférer à ces derniers un aspect nacré, préféré par le consommateur. Il a été constaté que ces agents nacrants ne confèrent pas aux cheveux des propriétés conditionnantes satisfaisantes.
Il existe donc un besoin d'une composition cosmétique notamment détergente, et en particulier un shampooing, qui présente un aspect nacré et/ou opacifié tout en permettant d'obtenir des performances cosmétiques acceptables des matières kératiniques, à savoir notamment les cheveux et le cuir chevelu.

Les tensioactifs amphotères alcoylamphohydroxyalkylsulfonates ont déjà été préconisés dans des compositions cosmétiques détergentes. Ils ont été décrits par exemple dans la demande de brevet WO99/36054.
Ces compositions de l'art antérieur présentent la caractéristique de ne pas avoir des propriétés cosmétiques satisfaisantes.

L'invention a donc pour but de proposer des compositions cosmétiques détergentes présentant des propriétés cosmétiques améliorées, en particulier le démêlage, le lissage, la souplesse ou la malléabilité des cheveux.

La, demanderesse a découvert, de façon surprenante, qu'il était possible de formuler des compositions cosmétiques de traitement des matières kératiniques, notamment des shampooings présentant un aspect nacré et/ou opacifié tout en ayant les propriétés esthétiques et cosmétiques recherchées, en utilisant dans ces compositions un agent nacrant et/ou opacifiant associés à au moins un tensioactif amphotère choisi parmi les composés alcoylamphohydroxyalkylsulfonates et leurs sels. En effet, il a été constaté que l'utilisation de ce tensioactif permettait d'obtenir sur les matières kératiniques, et notamment les cheveux des propriétés cosmétiques satisfaisantes, et en particulier permettait d'améliorer le démêlage ou le lissage des cheveux, et permettait d'apporter du volume, de la légèreté, de la douceur, de la souplesse ou de la malléabilité aux cheveux.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, au moins un tensioactif amphotère choisi parmi les composés alcoylamphohydroxyalkylsulfonates et leurs sels et au moins un agent nacrant et/ou opacifiant.

Un autre objet de l'invention concerne l'utilisation d'au moins un tensioactif amphotère choisi parmi les composés alcoylamphohydroxyalkylsulfonates et leurs sels dans, ou pour la fabrication d'une composition cosmétique comprenant un agent nacrant et/ou opacifiant.

Un autre objet de l'invention concerne un procédé de traitement des matières kératiniques, telles que les cheveux, caractérisé en ce qu'il consiste à appliquer sur lesdites matières des compositions cosmétiques selon l'invention.

L'invention a encore pour objet l'utilisation d'une composition selon l'invention pour améliorer le démêlage ou le lissage des cheveux, ou pour apporter du volume, de la légèreté, de la douceur, de la souplesse ou de la malléabilité aux cheveux.

Selon la présente invention, par matières kératiniques, on comprend les cheveux, les cils, les sourcils, la peau, les ongles, les muqueuses ou le cuir chevelu et plus particulièrement les cheveux.

Les différents objets de l'invention vont maintenant être détaillés. L'ensemble des significations et définitions des composés utilisés dans la présente invention données ci-dessous sont valables pour l'ensemble des objets de l'invention.

Les tensioactifs amphotères alcoylamphohydroxyalkylsulfonates peuvent avoir la formule suivante (I) : 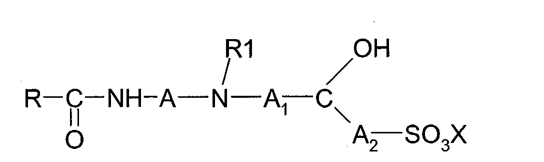 dans laquelle :
R désigne un radical hydrocarboné, saturé ou insaturé, linéaire ou ramifié comportant de 5 à 29 atomes de carbone.
R désigne de préférence un radical alkyle ou alkényle mono ou polyinsaturé comportant de 5 à 29 atomes de carbone et de préférence de 7 à 22 atomes de carbone et encore plus particulièrement de 9 à 17 atomes de carbone.
R1 désigne un radical hydroxyalkyle en C₁-C₄, de préférence hydroxyéthyle,
A, A1, A2 , identiques ou différents, désignent un radical alkylène divalent linéaire ou ramifié en C₁-C₁₀, de préférence en C₁-C₃
X désigne un atome d'hydrogène, un cation minéral ou organique tel que:
   Celui d'un métal alcalin (par exemple Na⁺, K⁺) , celui d'un métal alcalinoterreux (Mg²⁺, Ca²⁺) , l'ion NH₄⁺, les ions ammoniums issus des aminoacides basiques ou des amino-alcools.

Des composés préférés selon la présente invention sont des composés de formule (II) dans laquelle R désigne plus particulièrement un radical alkyle saturé, linéaire ou ramifié comportant de 7 à 29 atomes de carbone, de préférence de 7 à 22 atomes de carbone.

Lorsque X désigne un ion ammonium issu d'alcanolamine, celle-ci peut être la monoéthanolamine, la diéthanolamine, la triéthanolamine, l'amino-3 propanediol-1,2. Lorsque X désigne un ion ammonium issu d'amine, cette amine peut être un aminoacide basique tel que la lysine, l'arginine, la sarcosine, l'ornithine ou la citrulline.

De préférence, A= A2 et désigne -CH₂CH₂-.
De préférence, A1 désigne -CH₂-
De préférence X désigne Na⁺

Parmi les tensioactifs de formule (I), on peut citer plus particulièrement les sels de Cocoyl amphohydroxypropyl sulfonate et notamment le sel de sodium tel que le produit proposé sous la dénomination MIRANOL CSE par la société RHODIA CHIMIE ou les sels de palmitoyl amphohydroxypropyl sulfonate et notamment le sel de sodium tel que le produit proposé sous la dénomination MIRANOL CS par la société RHODIA CHIMIE.

Selon l'invention, le ou les tensioactifs amphotères choisis parmi les composés alcoylamphohydroxyalkylsulfonates et leurs sels peuvent représenter de 0,1 % à 30 % en poids, de préférence de 1% à 20% en poids et plus particulièrement de 1,5 % à 15 % en poids par rapport au poids total de la composition finale.

Les agents nacrants et/ou opacifiants de l'invention peuvent être choisis parmi :
i) les esters de polyols ayant au moins deux atomes de carbone et d'acides gras à chaîne longue, préférentiellement en C10-C30 et encore plus préférentiellement en C16-C22; Ces composés peuvent éventuellement être oxyéthylénés avec 2 à 15 moles d'oxyde d'éthylène. Les polyols ont de préférence de 2 à 4 atomes de carbone.
   Parmi ces composés, on peut citer notamment les monostéarates ou distéarates de polyéthylèneglycol et plus particulièrement le monostéarate d'éthylèneglycol et le distéarate d'éthylèneglycol.
ii) les alcanolamides d'acides gras à chaîne longue, préférentiellement en C10-C30 et encore plus préférentiellement en C16-C22, tels que le monoéthanolamide stéarique, le diéthanolamide stéarique, le monoisopropanolamide stéarique ou le stéarate de monoéthanolamide stéarique;
iii) les esters de monoalcools à chaîne longue (C10-C30) et d'acides gras à chaîne longue (C10-C30) comme le palmitate de cétyle;
iv) les éthers d'alcools gras à chaîne longue solides à une température inférieure ou égale à environ 30° C tels que par exemple les dialkyléthers de formule (II) :

   R-O-R' (II)

   dans laquelle R et R', identiques ou différents, désignent un radical alkyle, saturé ou insaturé, linéaire ou ramifié, comportant de 10 à 30 atomes de carbone et de préférence de 14 à 24 atomes de carbone, R et R' étant choisis de façon telle que le composé de formule (I) soit solide à une température inférieure ou égale à 30°C environ. Plus particulièrement, R et R' désignent un radical stéaryle. Ces composés peuvent notamment être préparés selon le procédé décrit dans la demande de brevet DE 41 27 230. Un distéaryléther utilisable dans le cadre de la présente invention, est commercialisé sous la dénomination CUTINA STE par la société HENKEL;
v) les esters à chaîne longue (C10-C30) d'alcanolamides à chaîne longue (C10-C30) tels que le distéarate stéaramide diéthanolamide ou le stéarate stéaramide monoéthanolamide;
vi) les alcools gras monochaînes ayant au moins 16 atomes de carbone et de préférence au moins 20 atomes de carbone tels que l'alcool béhénylique;
vii) les oxydes d'amine à chaîne longue (C10-C30), tels que les alkyl (C10-C30) diméthyl amine oxydes, comme par exemple le stéaryl diméthyl amine oxyde;
viii) les acides benzoïques N,N-dihydrocarbyl(C10-C30)amido, préférentiellement C12-C22 amido et leurs sels et particulièrement l'acide benzoïque N,N-di(C16-C18)amido commercialisé par la société STEPAN COMPANY; et
ix) les alcools ayant de 27 à 48 atomes de carbone et comportant un ou deux groupements éther et/ou thioéther ou sulfoxyde répondant à la formule (III) :

   Ra-X-[C2H3(OH)]-CH2-Y-Rb (III)

   dans laquelle Ra et Rb désignent indépendamment l'un de l'autre, des groupements linéaires en C12 à C24;
   X désigne un atome d'oxygène, un atome de soufre, un groupement sulfoxyde ou méthylène;
   Y désigne un atome d'oxygène, un atome de soufre, un groupement sulfoxyde ou méthylène;
   la somme du nombre d'atomes de carbone présents dans les groupements Ra et Rb a une valeur variant de 24 à 44 et de préférence de 28 à 40 inclus; lorsque X ou Y désigne sulfoxyde, Y ou X ne désigne pas soufre.
   Les composés de formule (III) utilisés de préférence conformément à l'invention, sont ceux pour lesquels X désigne oxygène, Y désigne méthylène, et Ra et Rb désignent des radicaux ayant 12 à 22 atomes de carbone, ces composés pouvant être préparés selon le brevet EP 457 688; Un composé particulièrement préféré est le 1-(hexadécyloxy)-2-octadécanol;
x) les oxydes de titane enrobés ou non, les micas et les mica titane; et
xi) les cyclodextrines;
   et leurs mélanges.

Les agents nacrants et/ou opacifiants sont préférentiellement choisis parmi les mono ou distéarate d'éthylène glycol, le distéaryl éther, l'alcool béhénylique et le 1 (hexadécyloxy)2-octadécanol et leurs mélanges.

Selon l'invention, l'agent nacrant et/ou opacifiant peut représenter de 0,2 % à 15 % en poids, de préférence de 0,5 % à 5 % en poids, par rapport au poids total de la composition finale.

Selon un mode de réalisation préféré, la composition comprend en outre au moins un polymère cationique.

De manière encore plus générale, au sens de la présente invention, l'expression "polymère cationique" désigne tout polymère contenant des groupements cationiques et/ou des groupements ionisables en groupements cationiques. Les polymères amphotères sont donc inclus dans cette famille de composés.

Les polymères cationiques utilisables conformément à la présente invention peuvent être choisis parmi tous ceux déjà connus en soi comme améliorant les propriétés cosmétiques, à savoir notamment ceux décrits dans la demande de brevet EP-A- 0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 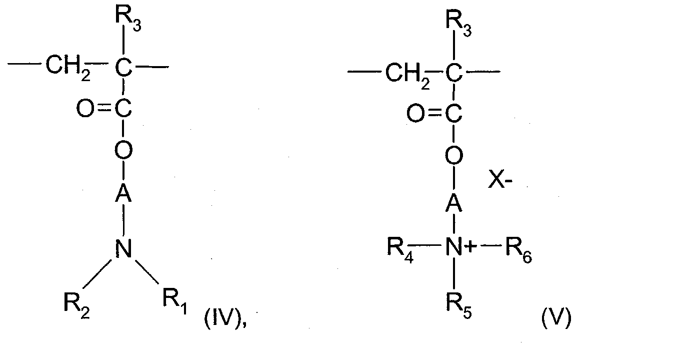 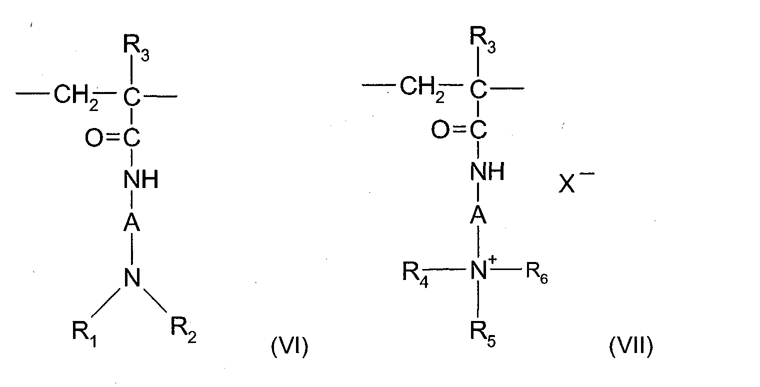 dans lesquelles:
   R₁ et R₂ , identiques ou différents, représentent hydrogène ou un groupe alkyle ayant de 1 à 6 atomes de carbone et de préférence méthyle ou éthyle;
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   X- désigne un anion dérivé d'un acide minéral ou organique tel que un anion méthylsulfate, éthylsulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
Ainsi, parmi ces copolymères de la famille (1), on peut citer :
- les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA ,
- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT® 734" ou "GAFQUAT® 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/ vinylpyrrolidone tel que le produit vendu sous la dénomination GAFFIX® VC 713 par la société ISP,
- les copolymères vinylpyrrolidone / méthacrylamidopropyl dimethylamine commercialisés notamment sous la dénomination STYLEZE® CC 10 par ISP.
- et les copolymères vinylpyrrolidone / méthacrylamide de diméthylaminopropyle quaternisé tel que le produit vendu sous la dénomination "GAFQUAT® HS 100" par la société ISP.

2) Les polysaccharides cationiques notamment les celluloses, les amidons et les gommes de galactomannanes cationiques. Parmi les polysaccharides cationiques, on peut citer plus particulièrement les éthers de cellulose comportant des groupements ammonium quaternaires, les copolymères de cellulose cationiques ou les celluloses greffées avec un monomère hydrosoluble d'ammonium quaternaire et les gommes de galactomannanes cationiques.

Les éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la société AMERCHOL. Ces polymères sont également définis dans le dictionnaire CTFA comme des ammonium quaternaires d'hydroxyéthylcellulose ayant réagi avec un époxyde substitué par un groupement triméthylammonium.

Les copolymères de cellulose cationiques ou les celluloses greffées avec un monomère hydrosoluble d'ammonium quaternaire, sont décrits notamment dans le brevet US 4 131 576. On peut notamment citer les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
Les produits commercialisés répondant à cette définition sont plus particulièrement les produits vendus sous la dénomination "CELQUAT® L 200" et "CELQUAT® H 100" par la société NATIONAL STARCH.

Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.

De tels produits sont commercialisés notamment sous les dénominations commerciales de JAGUAR® C13 S, JAGUAR® C 15, JAGUAR® C 17 ou JAGUAR® C162 par la société RHODIA CHIMIE.

On utilise également des amidons modifiées par un sel (par ex. chlorure) de 2,3-époxypropyl triméthylammonium tels que par exemple le produit dénommé Starch hydroxypropyltrimonium chloride selon la nomenclature INCI et commercialisé sous la dénomination SENSOMER CI-50 d'ONDEO.

(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;

(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(5) les polyaminoamides résultant de la condensation de polyalcoylènes polyamines avec des acides polycarboxyliques suivie d'une alcoylation par des agents bifonctionnels. On peut citer par exemple les polymères acide adipiquediacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces polyaminoamides, on peut citer plus particulièrement les polymères acide adipique/diméthylaminohydroxypropyl/diéthylène triamine vendus sous la dénomination "CARTARETINE® F, F4 ou F8" par la société SANDOZ.

(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.

Des polymères de ce type sont en particulier commercialisés sous la dénomination "HERCOSETT® 57" par la société HERCULES Inc. ou bien sous la dénomination de "PD 170" ou "DELSETTE® 101" par la société HERCULES dans le cas du copolymère d'acide adipique/époxypropyl/diéthylène-triamine.

(7) les cyclopolymères cationiques notamment d'alkyl diallyl amine ou de dialkyl diallyl ammonium tels que les homopolymères ou copolymères comportant comme motif principal de la chaîne des motifs répondant aux formules (VIII) ou (IX) : 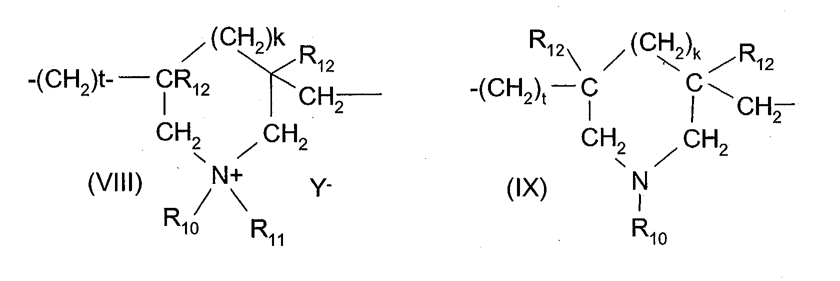 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 8 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
R₁₀ et R₁₁, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "MERQUAT® 100" par la société NALCO (et ses homologues de faibles masses moléculaires moyenne en poids) et les copolymères de chlorure de diallyldiméthylammonium et d'acrylamide commercialisés sous la dénomination "MERQUAT® 550".

(8) les polymères de diammonium quaternaires contenant des motifs récurrents répondant à la formule : 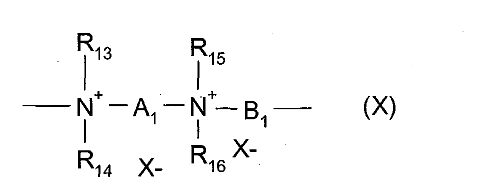 formule (X) dans laquelle :
R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n- dans lequel n est compris entre 1 et 100 et de préférence entre 1 et 50, et
dans lequel D désigne :
a) un reste de glycol de formule : -O-Z-O-, où Z désigne un radical hydrocarboné linéaire ou ramifié ou un groupement répondant à l'une des formules suivantes :

   - (CH2-CH2-O)x-CH2-CH2-

   - [CH2-CH(CH3)-O]y-CH2-CH(CH3)-

   où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

   - CH2-CH2-S-S-CH2-CH2- ;
d) un groupement uréylène de formule :

   -NH-CO-NH- ;

De préférence, X⁻ est un anion minéral ou organique monovalent tel qu'un halogénure (chlorure, bromure), un sulfate, ou un carboxylate (acétate, lactate, citrate).

Ces polymères ont une masse moléculaire moyenne en nombre généralement comprise entre 1.000 et 100.000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (XI) : 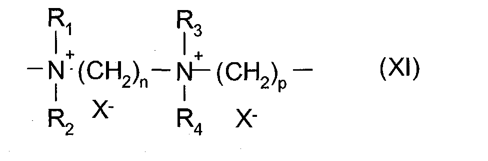 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
Un composé de formule (XI) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).

(9) les polymères de polyammonium quaternaires constitués de motifs de formule (XII): 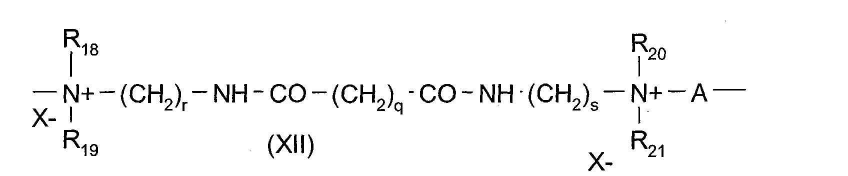 formule dans laquelle :
R₁₈, R₁₉, R₂₀ et R₂₁, identiques ou différents, représentent un atome d'hydrogène ou un radical méthyle, éthyle, propyle, β-hydroxyéthyle, β-hydroxypropyle ou - CH₂CH₂(OCH₂CH₂)ₚOH,
où p est égal à 0 ou à un nombre entier allant de 1 et 6, sous réserve que R₁₈, R₁₉, R₂₀ et R₂₁ ne représentent pas simultanément un atome d'hydrogène,
r et s, identiques ou différents, sont des nombres entiers allant de 1 et 6,
q est égal à 0 ou à un nombre entier allant de 1 et 34,
X⁻ désigne un anion tel qu'un halogènure,
A désigne un radical d'un dihalogénure ou représente de préférence - CH₂-CH₂-O-CH₂-CH₂-.
   De tels composés sont notamment décrits dans la demande de brevet EP-A-122 324.
   On peut par exemple citer parmi ceux-ci, les produits "MIRAPOL® A 15", "MIRAPOL® AD1 ", "MIRAPOL® AZ1" et "MIRAPOL® 175" vendus par la société MIRANOL.

(11) Les polymères quaternaires de vinylpyrrolidone et de vinylimidazole tels que les polyquaternium-11, polyquaternium-16, polyquaternium-44 notamment les produits commercialisés sous les dénominations LUVIQUAT® FC 905, FC 550, FC 370 et LUVIQUAT® CARE par la société B.A.S.F.

(11) Les polyamines comme le POLYQUART® H vendu par COGNIS, référencé sous le nom de « POLYETHYLENEGLYCOL (15) TALLOW POLYAMINE » dans le dictionnaire CTFA.

(12) Les polymères réticulés ou non réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les éthers de cellulose comportant des goupements ammonium quaternaires tels que les produits commercialisés sous la dénomination « JR 400 » par la société AMERCHOL, les cyclopolymères cationiques, en particulier les homopolymères de sel de diallyldiméthylammonium et les copolymères de sel de diallyldiméthylammonium et d'acrylamide en particulier les chlorures, commercialisés sous les dénominations « MERQUAT® 100 », « MERQUAT® 550 » et « MERQUAT® S » par la société NALCO, les gommes de guar modifiées par du chlorure de 2,3-époxypropyl triméthylammonium commercialisées par exemple sous la dénomination « JAGUAR C13S » par la société RHODIA CHIMIE et leurs mélanges.

Selon l'invention, le ou les polymères cationiques peuvent représenter de 0,001 % à 20 % en poids, de préférence de 0,01 % à 10% en poids et plus particulièrement de 0,1 à 3% en poids par rapport au poids total de la composition finale.

Selon un mode de réalisation particulierèment préféré, les compositions selon l'invention comprennent en outre au moins une silicone.

Les silicones utilisables conformément à l'invention peuvent être solubles ou insolubles dans la composition et en particulier être des polyorganosiloxanes insolubles dans la composition ; elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/ méthylakylsiloxane, tel que la "SILICONE VOLATILE FZ 3109" commercialisée par la société UNION CARBIDE, de structure chimique : 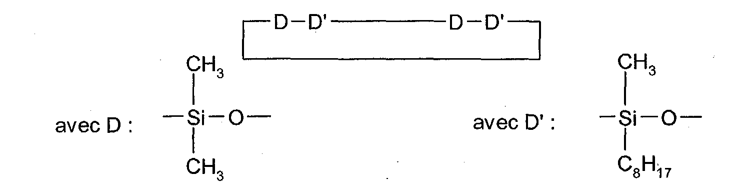 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32-TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
les huiles SILBIONE de la série 70 641 de RHONE POULENC ;
. les huiles des séries RHODORSIL 70 633 et 763 de RHONE POULENC ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
. les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334.
- des groupements acyloxyalkyle tels que par exemple les polyorganosiloxanes décrits dans le brevet US-A-4957732.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "AB I L S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 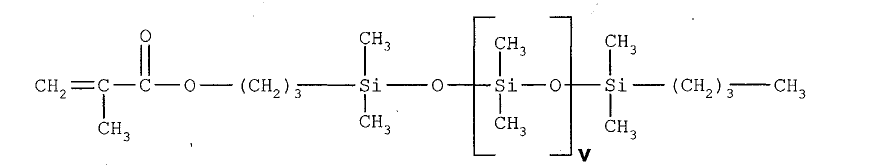 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions, de nanoémulsions ou de microémulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHONE POULENC, les polyalkylsiloxanes à groupements terminaux diméthylsilanol tels que les diméthiconol ou les polyalkylarylsiloxanes tels que l'huile SILBIONE 70641 V 200 commercialisée par la société RHONE POULENC ;
- la résine d'organopolysiloxane commercialisée sous la dénomination DOW CORNING 593 ;
- les polysiloxanes à groupements aminés tels que les amodiméthicones ou les triméthylsilylamodiméthicone ;

Selon l'invention, le ou les silicones peuvent représenter de 0,001 % à 20 % en poids, de préférence de 0,01 % à 10% en poids et plus particulièrement de 0,1 à 3% en poids par rapport au poids total de la composition finale.

Les compositions de l'invention contiennent en outre avantageusement au moins un agent tensioactif différents des tensioactifs amphotères selon l'invention qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

On utilise de préférence un agent tensioactif anionique choisi parmi les alkyl(C₁₂-C₁₄) sulfates de sodium, de magnésium ou d'ammonium, les alkyl (C₁₂-C₁₄)éthersulfates de sodium, de magnésium ou d'ammonium oxyéthylénés à 2,2 moles d'oxyde d'éthylène et le cocoyl iséthionate de sodium.

Parmi les tensioactifs anioniques, on préfère utiliser selon l'invention les sels d'alkylsulfates et d'alkyléthersufates et leurs mélanges.

### (ii) Tensioactif(s) non ionique(s):

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (1)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (2)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

### (iv) Tensioactif(s) cationique(s) :

Les tensioactifs cationiques peuvent être choisis parmi :
A) les sels d'ammonium quaternaires de la formule générale (XIII) suivante : 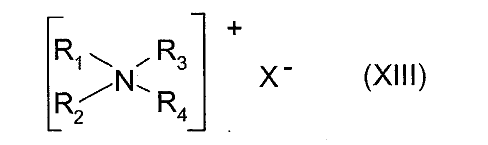 dans laquelle X est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate. , et
   i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R4 désigne un radical alkyle, linéaire ou ramifié, comportant de 16 à 30 atomes de carbone.
      De préférence le tensioactif cationique est un sel (par exemple chlorure) de béhényl triméthyl ammonium.
   ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
      R3 et R4, identiques ou différents, désignent un radical alkyle, linéaire ou ramifié, comportant de 12 à 30 atomes de carbone, ledit radical comprenant au moins une fonction ester ou amide.
      R3 et R4 sont notamment choisis parmi les radicaux alkyl(C₁₂-C₂₂)amido alkyle(C₂-C₆), alkyl(C₁₂-C₂₂)acétate ;
   De préférence le tensioactif cationique est un sel (par exemple chlorure) de stéaramidopropyl diméthyl (myristylacétate) ammonium.
B) - les sels d'ammonium quaternaire de l'imidazolinium, comme par exemple celui de formule (XIV) suivante : 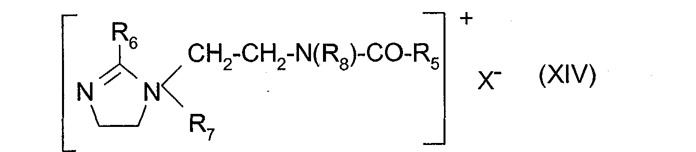 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) - les sels de diammonium quaternaire de formule (XV) : 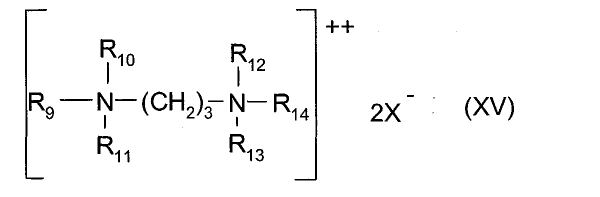 dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène
   ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire contenant au moins une fonction ester de formule (XVI ) suivante : 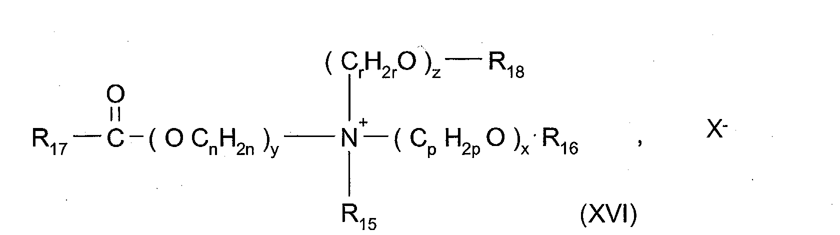 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
      - le radical 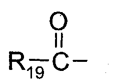
      - les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical 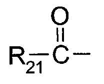
      - les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
   sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (XVI) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 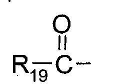
   - les radicaux méthyle, éthyle ou hydrocarbonés en C₁₄-C₂₂
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
   - le radical 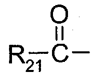
   - l'atome d'hydrogène ;

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques.

Le milieu cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.
De préférence , la composition comprend de 50 à 95 % en poids d'eau par rapport au poids total de la composition.

Les compositions cosmétiques selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

La composition de l'invention peut également contenir au moins un additif choisi parmi les épaississants, les agents antipelliculaires ou anti-séborrhéiques, les parfums, les électrolytes, les épaississants, les esters d'acides gras, les conservateurs, les filtres solaires siliconés ou non, les vitamines, les provitamines, les polymères anioniques ou non ioniques, les protéines, les hydrolysats de protéines, l'acide méthyl-18 eicosanoique, les hydroxyacides, le panthénol, les huiles végétales, animales, minérales ou de synthèse, les huiles fluorées ou perfluorées, les cires naturelles ou synthétiques, les composés de type céramide, les amines grasses, les acides gras et leurs dérivés, les alcools gras et leurs dérivés ainsi que les mélanges de ces différents composés et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

En particulier, les compositions selon l'invention sont des compositions détergentes telles que des shampooings, des gels-douche et des bains moussants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères et non ioniques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Lorsque la composition se présente sous la forme d'un après-shampooing éventuellement à rincer, elle contient avantageusement un tensioactif cationique, sa concentrentration généralement comprise entre 0,01 et 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

### EXEMPLE 1 :

On a réalisé deux compositions de shampooings, l'une conforme à l'invention (composition A) et l'autre comparative (composition B) :

| Composition | Invention (A) | Comparatif (B) |
|---|---|---|
| Cocoyl amphohydroxypropyl sulfonate de sodium, en solution aqueuse à 32% de matière active, proposé sous la dénomination MIRANOL CSE par la société RHODIA | 12 g M.A. | - |
| Cocoamidopropyl bétaïne en solution aqueuse à 30% de matière active | - | 12 g M.A. |
| Polydiméthylsiloxane de viscosité 500 000 cSt, vendue sous la dénomination Mirasil DM 500.000 par la société RHODIA | 1 g | 1 g |
| Mélange 1-(hexadécyloxy) 2-octadécanol / alcool cétylique | 2.5 g | 2.5 g |
| Monoisopropanolamide d'acides de coprah | 2 g | 2 g |
| Conservateurs | q.s. | q.s. |
| Parfum | q.s. | q.s. |
| Acide citrique ou soude q.s.p. | pH 7 | pH 7 |
| Eau déminéralisée qsp | 100 g | 100 g |

On effectue un shampooing en appliquant environ 6 g de la composition A sur une demi-tête de cheveux naturels préalablement mouillés. On fait mousser le shampooing puis on rince abondamment à l'eau. On sèche les cheveux avec un sèche-cheveux. On procède selon le même mode opératoire que ci-dessus avec la composition comparative B .
Les experts comparent les demi-têtes deux à deux.

Un panel d'experts a évalué l'aspect des cheveux séchés et a noté
- une plus grande facilité de démêlage,
- un plus grand lissage au toucher,
avec la composition conforme à l'invention.

Les cheveux traités avec la composition A sont significativement plus lisses et se démêlent plus facilement, que ceux traités avec la composition B.

### EXEMPLES 2 et 3

On a préparé les compositions de shampooing suivantes :

| Composition | Exemple 2 | Exemple 3 |
|---|---|---|
| Lauryléthersulfate de sodium (C12/C14 à 70/30) à 2.2 moles d'oxyde d'éthylène | 12 g M.A. | 15.5 g M.A. |
| Cocoyl amphohydroxypropyl sulfonate de sodium, en solution aqueuse à 32% de matière active, proposé sous la dénomination Miranol CSE par la société RHODIA | 1.8 g M.A. | 2.2 g M.A. |
| Hydroxyéthylcellulose réticulée par l'épichlorhydrine et quaternisée par la triméthylamine, vendue sous la dénomination JR 400 par la société AMERCHOL | 0.4 g | - |
| Gomme de guar modifiée par du chlorure de 2,3-époxypropyl triméthyl ammonium vendue sous la dénomination Jaguar C13 S par la société RHODIA | - | 0.1 g |
| Polydiméthylsiloxane de viscosité 500 000 cSt, vendu sous la dénomination Mirasil DM 500 000 par la société RHODIA | 1 g | - |
| Polydiméthylsiloxane de viscosité 300 000 cSt, vendu sous la dénomination DC 200 fluid 300 000 par la société DOW CORNING | - | 2.7 g |
| Mélange 1-(hexadécyloxy) 2-octadécanol / alcool cétylique | 2.5 g | - |
| Distéaryl éther | - | 1.5 g |
| Alcool béhénylique | - | 1.5 g |
| Mono-laurate de sorbitan oxyéthyléné à 4 moles d'oxyde d'éthylène, commercialisé sous la dénomination Tween 21 par la société UNIQEMA | 8 g | - |
| Monoisopropanolamide d'acides de coprah | 0.1 g | 1 g |
| Acide polyacrylique réticulé | 0.2 g | 0.2 g |
| Conservateurs | q.s. | q.s. |
| Parfum | q.s. | q.s. |
| Acide citrique q.s.p. | pH 5.3 | pH 7 |
| Eau déminéralisée q.s.p. | 100 g | 100 g |

Les cheveux lavés avec ces compositions sont lisses, souples et malléables.

### EXEMPLES 4 et 5

On a préparé les compositions de shampooing suivantes :

| Composition | Exemple 4 | Exemple 5 |
|---|---|---|
| Lauryléthersulfate de sodium (C12/C14 à 70/30) à 2.2 moles d'oxyde d'éthylène | 14 g M.A. | 11,2 g M.A. |
| Cocoyl amphohydroxypropyl sulfonate de sodium, en solution aqueuse à 32% de matière active, proposé sous la dénomination Miranol CSE par la société RHODIA | 3 g M.A. | 3 g M.A. |
| Hydroxyéthylcellulose réticulée par l'épichlorhydrine et quaternisée par la triméthylamine, vendue sous la dénomination JR 400 par la société AMERCHOL | | 0.8 g |
| Gomme de guar modifiée par du chlorure de 2,3-époxypropyl triméthyl ammonium | 0.18 g | |
| vendue sous la dénomination Jaguar C13 S par la société RHODIA | | |
| Polydiméthylsiloxane de viscosité 60 000 cSt, vendu sous la dénomination DC 200 fluid 60 000 par la société DOW CORNING | 2,5 g | |
| Mélange 1-(hexadécyloxy) 2-octadécanol / alcool cétylique | 2.5 g | - |
| Distéarate d'éthylèneglycol | - | 2 g |
| Monoisopropanolamide d'acides de coprah | | 3 g |
| Acide polyacrylique réticulé | | 0.2 g |
| Conservateurs | q.s. | q.s. |
| Parfum | q.s. | q.s. |
| Acide citrique ou NaOH q.s. | pH 7,5 | pH 5,3 |
| Eau déminéralisée q.s.p. | 100 g | 100 g |

Les cheveux lavés avec ces compositions sont lisses et se démêlent facilement.

## Revendications

1. Composition cosmétique, **caractérisée par le fait qu'**elle comprend, dans un milieu cosmétiquement acceptable, au moins un agent nacrant et/ou opacifiant et au moins un tensioactif amphotère choisi parmi les composés alcoylamphohydroxyalkylsulfonates et leurs sels.

2. Composition selon la revendication 1 , **caractérisée par le fait que** ledit tensioactif amphotère est choisi parmi les composés alcoylamphohydroxyalkylsulfonates ayant la formule suivante (I) : 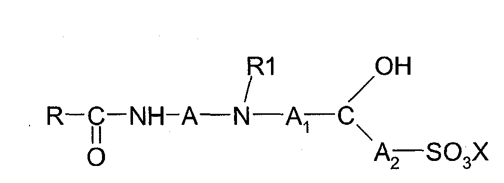 dans laquelle :
R désigne un radical hydrocarboné, saturé ou insaturé, linéaire ou ramifié comportant de 5 à 29 atomes de carbone.
R1 désigne un radical hydroxyalkyle en C₁-C₄, de préférence hydroxyéthyle,
A, A1, A2 , identiques ou différents, désignent un radical alkylène divalent linéaire ou ramifié en C₁-C₁₀, de préférence en C₁-C₃
X désigne un atome d'hydrogène, un cation minéral ou organique.

3. Composition selon la revendication 2, **caractérisée par le fait que** R désigne de préférence un radical alkyle ou alkényle mono ou polyinsaturé comportant de 5 à 29 atomes de carbone et de préférence de 7 à 22 atomes de carbone et encore plus particulièrement de 9 à 17 atomes de carbone.

4. Composition selon l'une quelconque des revendications 2 ou 3, **caractérisée par le fait que** A et A2 désignent -CH₂CH₂-.

5. Composition selon l'une quelconque des revendications 2 à 4, **caractérisée par le fait que** A1 désigne -CH₂-.

6. Composition selon l'une quelconque des revendications 2 à 5, **caractérisée par le fait que** le cation minéral ou organique est choisi parmi celui d'un métal alcalin ( par exemple Na⁺, K⁺) , celui d'un métal alcalinoterreux (Mg²⁺, Ca²⁺), l'ion NH₄⁺, les ions ammoniums issus des aminoacides basiques ou des amino-alcools.

7. Composition selon l'une quelconque des revendications 1 à 6, **caractérisée par le fait que** ledit tensioactif amphotère est choisi parmi les sels de Cocoyl amphohydroxypropyl sulfonate et les sels de palmitoyl amphohydroxypropyl sulfonate.

8. Composition selon l'une quelconque des revendications 1 à 7, **caractérisée en ce que** le ou les tensioactifs amphotères alcoylamphoallkylsulfonates et leurs sels sont présents à une concentration comprise entre 0,1 et 30 % en poids, de préférence entre 1 et 20% en poids par rapport au poids total de la composition.

9. Composition selon l'une quelconque des revendications précédentes, **caractérisée en ce que** l'agent nacrant et/ou opacifiant est choisi parmi :
i) les esters de polyols ayant au moins deux atomes de carbone et d'acides gras à chaîne longue en C10-C30;
ii) les alcanolamides d'acides gras à chaîne longue en C10-C30;
iii) les esters de monoalcools à chaîne longue et d'acides gras à chaîne longue en C10-C30;
iv) les éthers d'alcools gras à chaîne longue en C10-C30;
v) les esters à chaîne longue d'alcanolamides à chaîne longue en C10-C30;
vi) les alcools gras monochaînes ayant au moins 16 atomes de carbone;
vii) les oxydes d'amines à chaîne longue en C10-C30;
viii) les acides benzoïques N,N-dihydrocarbyl(C10-C30)amido et leurs sels;
x) les alcools ayant de 27 à 48 atomes de carbone et comportant un ou deux groupements éther et/ou thioéther ou sulfoxyde; et
x) les oxydes de titane enrobés ou non, les micas et les mica titane; et
xi) les cyclodextrines;
et leurs mélanges.

10. Composition selon l'une quelconque des revendications 1 à 9, **caractérisée en ce que** l'agent opacifiant et/ou nacrant est choisi parmi le monostéarate d'éthylèneglycol, le distéarate d'éthylène glycol, le distéaryl éther, l'alcool béhénylique et le 1 (hexadécyloxy)2-octadécanol et leurs mélanges.

11. Composition selon l'une quelconque des revendications 1 à 10, **caractérisée en ce que** l'agent opacifiant et/ou nacrant représente de 0,2% à 15 % en poids, de préférence de 0,5 % à 5 % en poids par rapport au poids total de la composition finale.

12. Composition selon l'une quelconque des revendications précédentes, **caractérisée en ce qu'**elle comprend en outre un polymère cationique.

13. Composition selon la revendication 12, **caractérisée par le fait que** les polymères cationiques sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

14. Composition selon l'une quelconque des revendications 13 ou 14, **caractérisée par le fait que** ledit polymère cationique est choisi parmi les éthers de cellulose comportant des goupements ammonium quaternaires, les homopolymères de sel de diallyldiméthylammonium et les copolymères de sel de diallyldiméthylammonium et d'acrylamide, les gommes de guar modifiées par du chlorure de 2,3-époxypropyl triméthylammonium et leurs mélanges.

15. Composition selon l'une quelconque des revendications 12 à 14, **caractérisée en ce que** le polymère cationique est présent à une concentration comprise entre 0,001 % et 20 % en poids par rapport au poids total de la composition, de préférence entre 0,01 % et 10 % en poids.

16. Composition selon l'une quelconque des revendications 1 à 15, **caractérisée par le fait qu'**elle comprend en outre au moins une silicone.

17. Composition selon la revendication 16, **caractérisée par le fait que** les silicones sont des polyorganosiloxanes non volatils choisis parmi les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

18. Composition selon l'une quelconque des revendications 16 à 17, **caractérisée par le fait que** les silicones sont choisis parmi les polyalkylsiloxanes à groupements terminaux triméthylsilyle, les polyalkylsiloxanes à groupements terminaux diméthylsilanol, les polyalkylarylsiloxanes, les silicones aminées.

19. Composition selon l'une quelconque des revendications 16 à 18, **caractérisée en ce que** ladite silicone est présente à une concentration comprise entre 0,001 % et 20 % en poids par rapport au poids total de la composition, de préférence entre 0,01 % et 10 % en poids.

20. Composition selon l'une quelconque des revendications précédentes, **caractérisée par le fait qu'**elle comprend en outre au moins un agent tensioactif additionnel choisi parmi les tensioactifs anioniques, non ioniques, amphotères, cationiques et leurs mélanges.

21. Composition selon la revendication 20, **caractérisée par le fait que** le ou les agents tensioactifs additionnels sont présents à une concentration comprise entre 0,1% et 60% en poids, de préférence entre 3% et 40% en poids, et encore plus préférentiellement entre 5% et 30% en poids, par rapport au poids total de la composition.

22. Composition selon l'une quelconque des revendications 1 à 21, **caractérisée par le fait que** la composition contient au moins un additif choisi parmi les épaississants, les agents antipelliculaires ou anti-séborrhéiques, les parfums, les électrolytes, les épaississants, les esters d'acides gras, les conservateurs, les filtres solaires siliconés ou non, les vitamines, les provitamines, les polymères anioniques ou non ioniques, les protéines, les hydrolysats de protéines, l'acide méthyl-18 eicosanoique, les hydroxyacides, le panthénol, les huiles végétales, animales, minérales ou de synthèse, les huiles fluorées ou perfluorées, les cires naturelles ou synthétiques, les composés de type céramide, les amines grasses, les acides gras et leurs dérivés, les alcools gras et leurs dérivés ainsi que les mélanges de ces différents composés.

23. Composition selon l'une quelconque des revendications précédentes, **caractérisée par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavante pour le corps.

24. Utilisation d'une composition telle que définie dans l'une quelconque des revendications 1 à 23 pour améliorer le démêlage ou le lissage des cheveux , ou pour apporter du volume, de la légèreté, de la douceur, de la souplesse, de la malléabilité aux cheveux.

25. Utilisation d'un tensioactif amphotère alcoylampho hydroxyalkyl sulfonate ou ses sels dans une composition cosmétique contenant au moins un agent nacrant et/ou opacifiant pour améliorer le démêlage ou le lissage des cheveux, ou pour apporter du volume, de la légèreté, de la douceur, de la souplesse ou de la malléabilité aux cheveux.

26. Procédé de traitement des matières kératiniques, telles que les cheveux, **caractérisé en ce qu'**il consiste à appliquer sur lesdites matières une composition cosmétique selon l'une des revendications 1 à 23, puis à effectuer éventuellement un rinçage.
